# EUROPEAN PATENT APPLICATION

(11) **EP 4 285 848 A1**
(43) Date of publication of application: **06.12.2023**
(21) Application number: 23172804.9
(22) Date of filing: 11.05.2023
(51) Int. Cl.: A61B 17/70

(54) **SPINAL IMPLANT SYSTEM**

(30) Priority: 16.05.2022 US 202217744950
(71) Applicant: Warsaw Orthopedic, Inc., Warsaw, IN 46582 (US)
(72) Inventor: RIDING, Shane Lars, Bartlett, TN (US); PREVOST, Julien J., Memphis, TN (US); ITALIAIE, Christel, Memphis, TN (US); REZACH, William Alan, Covington, TN (US)
(74) Representative: Viering, Jentschura & Partner mbB Patent- und Rechtsanwälte

(57) **Abstract**

A spinal implant system includes a first bone fastener including a first head and a first shaft. The first head including a medial rod receiver offset from the first shaft. A second bone fastener is provided including a second head and a second shaft. The second head including a lateral rod receiver offset from the second shaft. The shafts are configured for fixation with vertebral tissue in alignment along a selected trajectory of the vertebral tissue. Surgical instruments, implants and methods are disclosed.

## Description

### TECHNICAL FIELD

The present disclosure generally relates to medical devices for the treatment of musculoskeletal disorders, and more particularly to a spinal implant system and a method for treating a spine.

### BACKGROUND

Spinal pathologies and disorders such as scoliosis and other curvature abnormalities, kyphosis, degenerative disc disease, disc herniation, osteoporosis, spondylolisthesis, stenosis, tumor and fracture may result from factors including trauma, disease and degenerative conditions caused by injury and aging. Spinal disorders typically result in symptoms including deformity, pain, nerve damage, and partial or complete loss of mobility.

Non-surgical treatments, such as medication, rehabilitation and exercise can be effective, however, may fail to relieve the symptoms associated with these disorders. Surgical treatment of these spinal disorders includes correction, fusion, fixation, discectomy, laminectomy and implantable prosthetics. As part of these surgical treatments, spinal constructs including vertebral rods are often used to provide stability to a treated region. Rods redirect stresses away from a damaged or defective region while healing takes place to restore proper alignment and generally support vertebral members. During surgical treatment, one or more rods and bone fasteners can be delivered to a surgical site. The rods may be attached via the fasteners to the exterior of two or more vertebral members. This disclosure describes an improvement over these prior technologies.

### SUMMARY

In one embodiment, a spinal implant system is provided. The spinal implant system includes a first bone fastener including a first head and a first shaft. The first head including a medial rod receiver offset from the first shaft. A second bone fastener is provided including a second head and a second shaft. The second head including a lateral rod receiver offset from the second shaft. The shafts are configured for fixation with vertebral tissue in alignment along a selected trajectory of the vertebral tissue. In some embodiments, surgical instruments, implants and methods are disclosed.

In one embodiment, the spinal implant system includes a first bone fastener including a first head and a first shaft. The first head including a medial rod receiver offset from the first shaft. A second bone fastener is provided including a second head and a second shaft. The second head including a medial rod receiver offset from the second shaft. A third bone fastener is provided including a third head and a third shaft. The third head including a lateral rod receiver offset from the third shaft. A fourth bone fastener is provided including a fourth head and a fourth shaft. The fourth head including a lateral rod receiver offset from the fourth shaft. The shafts configured for fixation with vertebral tissue in alignment along a selected trajectory of the vertebral tissue. A first spinal rod is configured for disposal within the medial rod receivers, and a second spinal rod is configured for disposal within the lateral rod receivers.

In one embodiment, a method for treating a spine is provided. The method comprising the steps of: fixing a first shaft of a first bone fastener with vertebral tissue, the first bone fastener including a first head including a medial rod receiver offset from the first shaft; fixing a second shaft of a second bone fastener with the vertebral tissue in alignment with the first shaft along a selected trajectory of the vertebral tissue, the second bone fastener including a second head including a lateral rod receiver offset from the second shaft; disposing a first spinal rod within the medial rod receiver; and disposing a second spinal rod within the lateral rod receiver.

### BRIEF DESCRIPTION OF THE DRAWINGS

The present disclosure will become more readily apparent from the specific description accompanied by the following drawings, in which:
FIG. 1 is a perspective view of components of one embodiment of a system in accordance with the principles of the present disclosure;
FIG. 2 is a perspective view of components of the system shown in FIG. 1;
FIG. 3 is a perspective view of components of the system shown in FIG. 1;
FIG. 4 is a perspective view of components of the system shown in FIG. 1;
FIG. 5 is a perspective view of components of the system shown in FIG. 1;
FIG. 6 is a perspective view of components of the system shown in FIG. 1;
FIG. 7 is a cross section view of the components shown in FIG. 5;
FIG. 8 is a cross section view of the components shown in FIG. 6;
FIG. 9 is a perspective view of components of the system shown in FIG. 1;
FIG. 10 is a perspective view of components of the system shown in FIG. 1;
FIG. 11 is a perspective view of components of the system shown in FIG. 1;
FIG. 12 is a cross section view of the components shown in FIG. 11; and
FIG. 13 is a perspective view of components of the system shown in FIG. 1 with parts separated.

### DETAILED DESCRIPTION

The exemplary embodiments of the surgical system and related methods of use disclosed are discussed in terms of medical devices for the treatment of musculoskeletal disorders and more particularly, in terms of a surgical system and method for treatment of a spine disorder. In some embodiments, a spinal implant system is provided that includes a spinal construct having a plurality of spinal rods employed with a single side of vertebrae. In some embodiments, the spinal construct is fixed with vertebral tissue along a single bone fastener trajectory. In some embodiments, the spinal construct is fixed with vertebral tissue along a single bone fastener trajectory employing offset rod receivers. In some embodiments, the systems and methods of the present disclosure are employed with treatment of a spine disorder, for example, relating to a cervical, thoracic, lumbar and/or sacral region of a spine.

In some embodiments, the present spinal implant system includes a spinal construct having one or more bone fasteners configured to connect a plurality of spinal rods with a single side of vertebrae. In some embodiments, the bone fasteners each include a shaft fixed with vertebral tissue and a head including a rod receiver offset from the shaft such that the spinal construct is fixed with the vertebral tissue along a single bone fastener trajectory. In some embodiments, the spinal construct is fixed with the vertebral tissue along a single bone fastener trajectory and includes a plurality of spinal rods, for example, a medial rod and a lateral rod, employed with a single side of vertebrae. In some embodiments, the present spinal implant system provides a spinal construct configuration as described herein that avoids employing multiple bone fastener trajectories along a single side of vertebrae.

In some embodiments, the present spinal implant system includes a spinal construct having a plurality of bone fasteners including offset heads employed with dual spinal rods along a single side of vertebrae. In some embodiments, the offset heads facilitate disposal of the spinal rods along a single fastener trajectory. In some embodiments, the spinal construct includes one or more modular bone fasteners having interchangeable heads and/or shafts. In some embodiments, the dual spinal rods are configured for engagement with the plurality of bone fasteners such that the dual spinal rods are seated simultaneously with each of the plurality of bone fasteners. In some embodiments, the surgical system is configured to improve a surgeon's ability to utilize dual spinal rods in a single construct along a single side of vertebrae in a spinal fixation procedure along a single fastener trajectory. In some embodiments, the present surgical system increases the number of surgeons that can employ a dual rod construct.

In some embodiments, the present surgical system described herein is implemented in spinal reconstruction procedures, for example, corpectomies and/or osteotomies. In some embodiments, one or more of the spinal rods include a 4.75 to 6.0 mm diameter. In some embodiments, one or more of the spinal rods are configured for reduction relative to vertebrae of a patient. In some embodiments, the spinal construct includes one or more rod receivers configured for engagement with a setscrew for locking a spinal rod with the receiver. In some embodiments, the setscrew is configured to provisionally lock with a spinal rod to facilitate spinal rod seating with the rod receiver. In some embodiments, the bone fasteners are configured to increase or decrease medial lateral offset of the spinal rods. In some embodiments, the shaft of the bone fastener includes an axis and the rod receiver of the bone fastener includes an axis. In some embodiments, the axis of the rod receiver is angled relative to the shaft axis. In some embodiments, the offset rod receiver enables a single trajectory to be employed for a dual rod construct. In some embodiments, a single trajectory of the bone fasteners enable ease of alignment of the rod receivers simultaneously. In some embodiments, a single trajectory of the bone fasteners facilitates rod bending and/or contouring.

In some embodiments, the present surgical system facilitates alignment of the shafts of the plurality of bone fasteners in a single trajectory that is selected by the surgeon. In some embodiments, the plurality of heads including the rod receivers offset from the shafts are disposed in alternating orientations with the dual rods to provide space for the rods. In some embodiments, the system includes spinal rod multi axial screw heads and/or standard bone fastener heads in conjunction with the offset receivers described above.

In some embodiments, one or all of the components of the surgical system may be disposable, peel-pack, pre-packed sterile devices. One or all of the components of the system may be reusable. The system may be configured as a kit with multiple sized and configured components.

In some embodiments, the surgical system of the present disclosure may be employed to treat spinal disorders such as, for example, degenerative disc disease, disc herniation, osteoporosis, spondylolisthesis, stenosis, scoliosis and other curvature abnormalities, kyphosis, tumor and fractures. In some embodiments, the surgical system of the present disclosure may be employed with other osteal and bone related applications, including those associated with diagnostics and therapeutics. In some embodiments, the disclosed surgical system may be alternatively employed in a surgical treatment with a patient in a prone or supine position, and/or employ various surgical approaches to the spine, including anterior, posterior, posterior mid-line, direct lateral, postero-lateral, and/or antero-lateral approaches, and in other body regions. The surgical system of the present disclosure may also be alternatively employed with procedures for treating the lumbar, cervical, thoracic, sacral and pelvic regions of a spinal column. The surgical system of the present disclosure may also be used on animals, bone models and other non-living substrates, such as, for example, in training, testing and demonstration.

The surgical system of the present disclosure may be understood more readily by reference to the following detailed description of the embodiments taken in connection with the accompanying drawing figures, which form a part of this disclosure. It is to be understood that this application is not limited to the specific devices, methods, conditions or parameters described and/or shown herein, and that the terminology used herein is for the purpose of describing particular embodiments by way of example only and is not intended to be limiting. In some embodiments, as used in the specification and including the appended claims, the singular forms "a," "an," and "the" include the plural, and reference to a particular numerical value includes at least that particular value, unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value. Similarly, when values are expressed as approximations, by use of the antecedent "about," it will be understood that the particular value forms another embodiment. It is also understood that all spatial references, such as, for example, horizontal, vertical, top, upper, lower, bottom, left and right, are for illustrative purposes only and can be varied within the scope of the disclosure. For example, the references "upper" and "lower" are relative and used only in the context to the other, and are not necessarily "superior" and "inferior".

As used in the specification and including the appended claims, "treating" or "treatment" of a disease or condition refers to performing a procedure that may include administering one or more drugs to a patient (human, normal or otherwise or other mammal), employing implantable devices, and/or employing instruments that treat the disease, such as, for example, microdiscectomy instruments used to remove portions bulging or herniated discs and/or bone spurs, in an effort to alleviate signs or symptoms of the disease or condition. Alleviation can occur prior to signs or symptoms of the disease or condition appearing, as well as after their appearance. Thus, treating or treatment includes preventing or prevention of disease or undesirable condition (e.g., preventing the disease from occurring in a patient, who may be predisposed to the disease but has not yet been diagnosed as having it). In addition, treating or treatment does not require complete alleviation of signs or symptoms, does not require a cure, and specifically includes procedures that have only a marginal effect on the patient. Treatment can include inhibiting the disease, e.g., arresting its development, or relieving the disease, e.g., causing regression of the disease. For example, treatment can include reducing acute or chronic inflammation; alleviating pain and mitigating and inducing regrowth of new ligament, bone and other tissues; as an adjunct in surgery; and/or any repair procedure. In some embodiments, as used in the specification and including the appended claims, the term "tissue" includes soft tissue, ligaments, tendons, cartilage and/or bone unless specifically referred to otherwise.

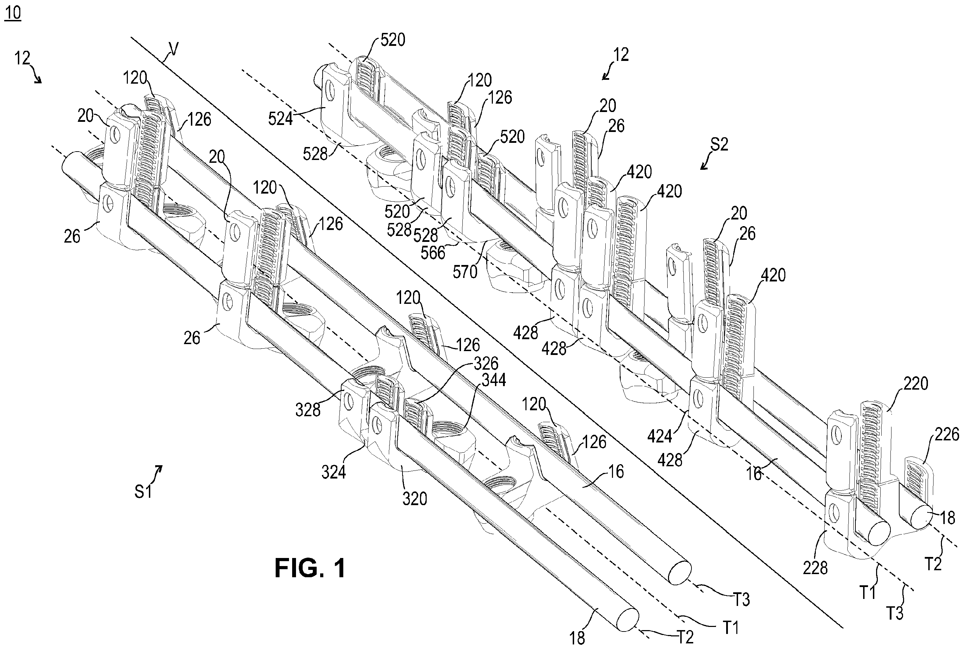

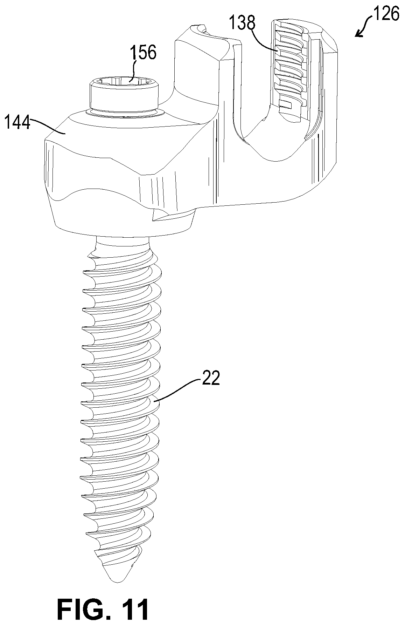

The following discussion includes a description of a surgical system including a spinal construct, related components and methods of employing the surgical system in accordance with the principles of the present disclosure. Alternate embodiments are also disclosed. Reference is made in detail to the exemplary embodiments of the present disclosure, which are illustrated in the accompanying figures. Turning to FIGS. 1-13 there are illustrated components of a surgical system, such as, for example, a spinal implant system 10.

The components of spinal implant system 10 can be fabricated from biologically acceptable materials suitable for medical applications, including metals, synthetic polymers, ceramics and bone material and/or their composites. For example, the components of spinal implant system 10, individually or collectively, can be fabricated from materials such as stainless steel alloys, aluminum, commercially pure titanium, titanium alloys, Grade 5 titanium, super-elastic titanium alloys, cobalt-chrome alloys, superelastic metallic alloys (e.g., Nitinol, super elasto-plastic metals, such as GUM METAL^{®}), ceramics and composites thereof such as calcium phosphate (e.g., SKELITE^{™}), thermoplastics such as polyaryletherketone (PAEK) including polyetheretherketone (PEEK), polyetherketoneketone (PEKK) and polyetherketone (PEK), carbon-PEEK composites, PEEK-BaSO₄ polymeric rubbers, polyethylene terephthalate (PET), fabric, silicone, polyurethane, silicone-polyurethane copolymers, polymeric rubbers, polyolefin rubbers, hydrogels, semi-rigid and rigid materials, elastomers, rubbers, thermoplastic elastomers, thermoset elastomers, elastomeric composites, rigid polymers including polyphenylene, polyamide, polyimide, polyetherimide, polyethylene, epoxy, bone material including autograft, allograft, xenograft or transgenic cortical and/or corticocancellous bone, and tissue growth or differentiation factors, partially resorbable materials, such as, for example, composites of metals and calcium-based ceramics, composites of PEEK and calcium based ceramics, composites of PEEK with resorbable polymers, totally resorbable materials, such as, for example, calcium based ceramics such as calcium phosphate, tri-calcium phosphate (TCP), hydroxyapatite (HA)-TCP, calcium sulfate, or other resorbable polymers such as polyaetide, polyglycolide, polytyrosine carbonate, polycaroplaetohe and their combinations.

Various components of spinal implant system 10 may have material composites, including the above materials, to achieve various desired characteristics such as strength, rigidity, elasticity, compliance, biomechanical performance, durability and radiolucency or imaging preference. The components of spinal implant system 10, individually or collectively, may also be fabricated from a heterogeneous material such as a combination of two or more of the above-described materials. The components of spinal implant system 10 may be monolithically formed, integrally connected or include fastening elements and/or instruments, as described herein.

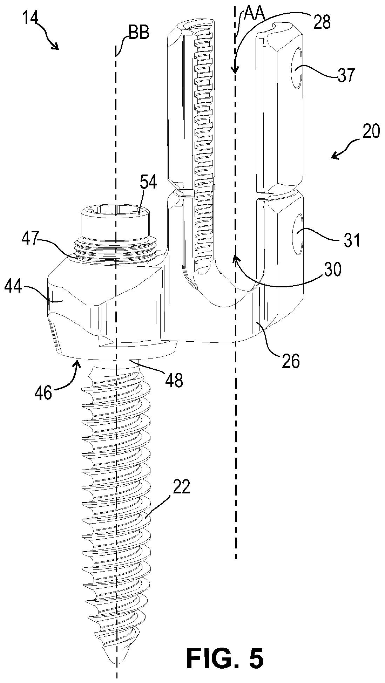

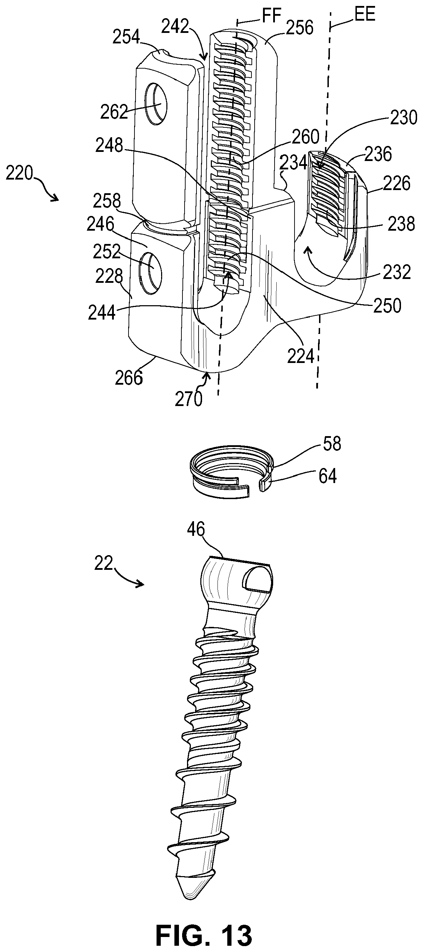

Spinal implant system 10 includes a spinal construct 12 including one or more components for disposal along a side of vertebrae of a patient to provide strength and facilitate surgical treatment. In some embodiments, spinal construct 12 includes multiple spinal rods connected with bone fasteners fixed with tissue along a single bone fastener trajectory in a configuration that improves construct strength and resists and/or prevents spinal rod failure, as described herein. In some embodiments, spinal construct 12 includes a plurality of bone fasteners, for example bone fasteners 14, as described herein, that may include heads with offset receivers, for example, heads 20 (as shown in FIG. 5) and/or heads 220 (as shown in FIG. 13) that connect a plurality of spinal rods with a single side of vertebrae. In some embodiments, spinal construct 12 includes a plurality of spinal rods including a medial rod and a lateral rod, for example a dual rod construct fastened to a single side of vertebrae to increase construct strength in a spinal fixation treatment.

For example, spinal construct 12 includes bone fastener 14, as shown in FIGS. 1-8. Referring to FIG. 5, fastener 14 includes head 20 and a shaft 22 for fixation with vertebral tissue. Shaft 22 is aligned along a selected trajectory T1, as shown in FIG. 1 and described herein. In some embodiments, all or a portion of the selected trajectory T1 includes and/or is disposed along a straight line, a linear axis, angled and/or a curved pathway that shaft 22 is disposed relative to vertebral tissue. In some embodiments, the selected trajectory T1 is disposed along a sagittal, coronal, transverse, dorsal, medial, and/or lateral orientation relative to the vertebral tissue. In some embodiments, head 20 is selected from a plurality of alternate heads 20 and is configured for disposal with shaft 22 such that shaft 22 is interchangeable with the plurality of alternate heads 20.

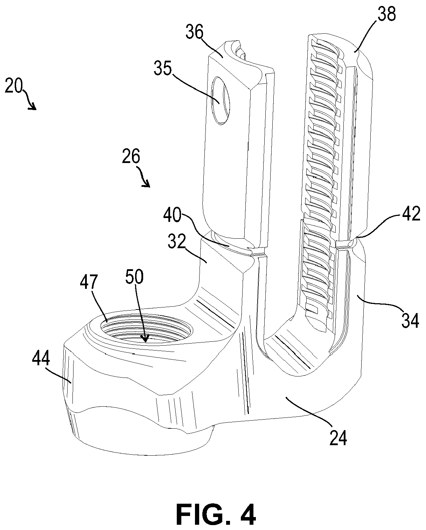

Head 20 includes a body 24, as shown in FIG. 4. Body 24 includes a surface that defines a rod receiver 26 configured to receive a spinal rod, for example a spinal rod 16 and/or a spinal rod 18 shown in FIG. 1. In some embodiments, receiver 26 is configured to receive a spinal rod that is oriented medially relative to a spine and/or a spinal rod that is oriented laterally relative to a spine. Referring to FIG. 5, receiver 26 is disposed in an offset orientation relative to shaft 22. Receiver 26 defines a longitudinal axis AA and is aligned along a trajectory T2 or a trajectory T3 that is offset from the selected trajectory T1 of shaft 22, as shown in FIG. 1 and described herein. In some embodiments, all or a portion of trajectory T2 and/or T3 includes and/or is disposed along a straight line, a linear axis, angled and/or a curved pathway that receiver 26 is disposed relative to vertebral tissue. In some embodiments, the trajectories T2 and/or T3 are disposed along a sagittal, coronal, transverse, dorsal, medial, and/or lateral orientation relative to the vertebral tissue. In some embodiments, all or only a portion of receiver 26 may have alternate cross section configurations, such as, for example, closed, V-shaped, co-shaped, W-shaped, oval, oblong triangular, square, polygonal, irregular, uniform, non-uniform, offset, staggered, and/or tapered.

Referring to FIG. 5, an inner surface of receiver 26 defines a cavity 28 for disposal of spinal rod 16 or spinal rod 18, as shown in FIG. 1. Cavity 28 extends along longitudinal axis AA and includes a transverse passageway 30. In some embodiments, cavity 28 may have various cross section configurations, such as, for example, oval, oblong, triangular, rectangular, square, polygonal, irregular, uniform, non-uniform, variable and/or tapered. In some embodiments, all or a portion of the inner surface is smooth, rough, arcuate, undulating, mesh, porous, semi-porous, dimpled and/or textured.

Receiver 26 includes spaced apart arms 32, 34, as shown in FIG. 4. An inner surface of arms 32, 34 defines threads 33 for engagement with threads of a setscrew (not shown). An outer surface of arm 34 defines a recess 31, as shown in FIG. 5. Recess 31 is configured to receive an insertion tool, compression instrument and/or surgical instruments for reducing spinal rod 16 or spinal rod 18 with receiver 26 and/or to manipulate fastener 14. In some embodiments, all or a portion of the recess 31 is smooth, rough, arcuate, undulating, mesh, porous, semi-porous, dimpled and/or textured to assist in engagement with the surgical instrument.

Arm 32 includes an extended reduction tab 36 and arm 34 includes an extended reduction tab 38, as shown in FIG. 4. Reduction tabs 36, 38 facilitate reduction of spinal rod 16 or spinal rod 18, as described herein, with receiver 26. Reduction tabs 36, 38 include break off portions 40, 42. In some embodiments, reduction tabs 36, 38 fracture and separate at break off portions 40, 42 at a predetermined force or torque limit, which may be in a range of approximately 2 Newton meters (N-m) to 8 Nm. Reduction tab 36 includes a recess 35 and reduction tab 38 includes a recess 37 (as shown in FIG. 5) configured to receive an insertion tool, compression instrument and/or surgical instruments for reducing spinal rod 16 or spinal rod 18 with receiver 26 and/or to manipulate fastener 14. In some embodiments, all or a portion of the recesses 35, 37 are smooth, rough, arcuate, undulating, mesh, porous, semi-porous, dimpled and/or textured to assist in engagement with the surgical instrument. In some embodiments, reduction tabs 36, 38 and arms 32, 34 may have the same or alternate cross section configurations, may be fabricated from a homogenous material or heterogeneously fabricated from different materials, and/or alternately formed of a material having a greater degree, characteristic or attribute of plastic deformability, frangible property and/or break away quality to facilitate fracture and separation of reduction tabs 36, 38 from arms 32, 34.

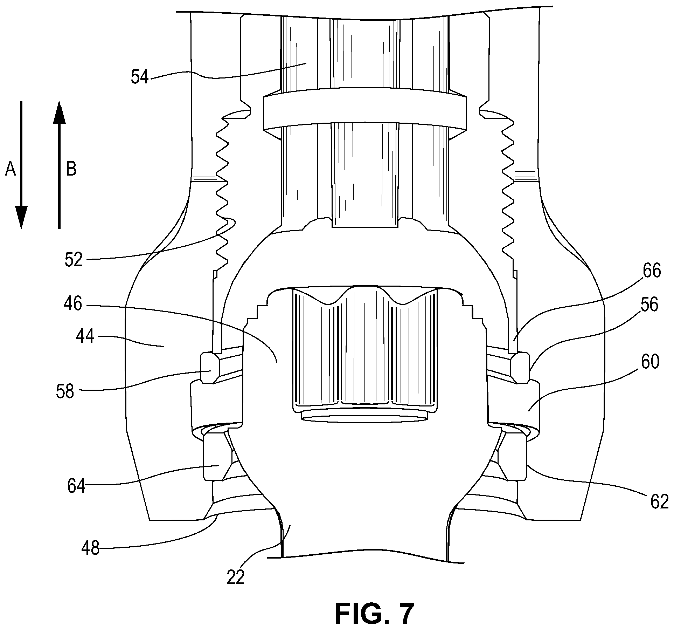

Body 24 includes a surface that defines a mount 44, as shown in FIG. 4. Mount 44 is configured for engagement with a head 46 of shaft 22, as shown in FIGS. 5 and 7. An outer surface of mount 44 defines an opening 47, an opening 48 and a longitudinal axis BB, as shown in FIGS. 4 and 5. An inner surface of mount 44 defines a channel 50 disposed between openings 47, 48. Head 46 of shaft 22 is disposed with opening 48 and channel 50. In some embodiments, channel 50 may have various cross section configurations, such as, for example, oval, oblong, triangular, rectangular, square, polygonal, irregular, uniform, non-uniform, variable and/or tapered.

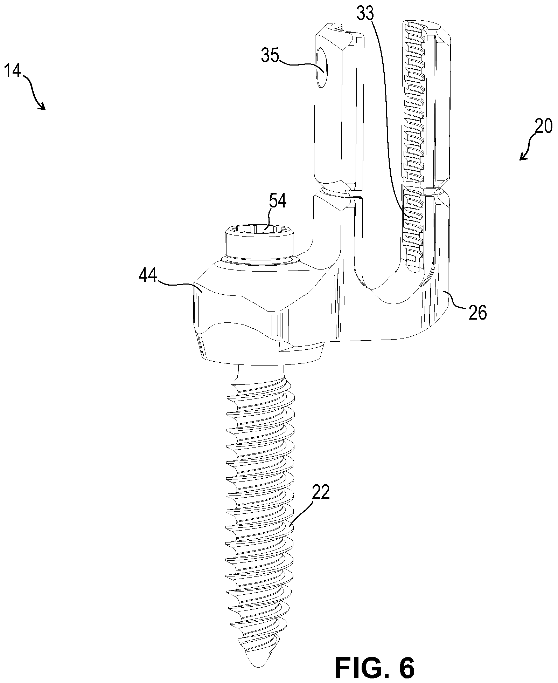

The inner surface of mount 44 defines a plurality of threads 52 for engagement with a threaded locking element 54, as shown in FIG. 7. Locking element 54 is movable relative to mount 44 to dispose shaft 22 in a non-locked orientation (FIG. 5) and a locked orientation (FIG. 6). Referring to FIG. 7, the inner surface of mount 44 defines a circumferential upper groove 56 for disposal of a resilient member, for example, a ring 58. Ring 58 is contractable in upper groove 56 and is configured for engagement with an end 66 of locking element 54. Ring 58 includes a circumference that defines an opening, for example, a gap. In some embodiments, the gap is sized such that the gap has a length that is less than the height or the thickness of ring 58. In some embodiments, the gap is sized to allow ring 58 to contract circumferentially and to allow ring 58 to translate through channel 50 of mount 44. In some embodiments, upon disposal of ring 58 with upper groove 56, surfaces of upper groove 56 resist and/or prevent axial translation of ring 58 relative to axis longitudinal axis BB. The inner surface of mount 44 also defines an expansion groove 60.

The inner surface of mount 44 further defines a circumferential lower groove 62 for disposal of a resilient member, for example, a ring 64. Ring 64 is expandable in expansion groove 60. Ring 64 includes a circumference that defines an opening, for example, a gap. In some embodiments, the gap is sized such that the gap has a length that is less than the height and the thickness of ring 64. In some embodiments, the gap is sized to allow ring 64 to contract circumferentially and allow ring 64 to translate through channel 50 of mount 44. In some embodiments, upon disposal of ring 64 with lower groove 62, surfaces of lower groove 62 resist and/or prevent axial translation of ring 64 relative to axis longitudinal axis BB. In some embodiments, rings 58, 64 facilitate manual engagement/connection of mount 44 and shaft 22 such that shaft 22 is attached with mount 44 in a non-instrumented snap-fit assembly, as described herein.

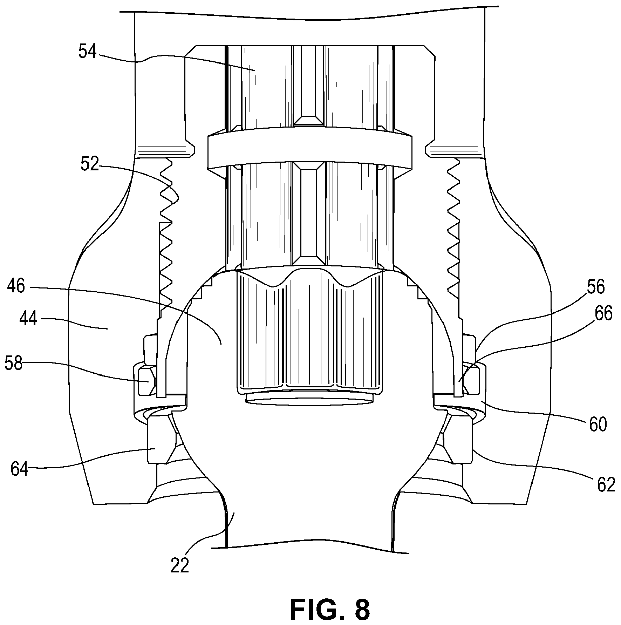

In the non-locked orientation, as shown in FIGS. 5 and 7, locking element 54 does not fully translate through channel 50 and end 66 does not translate ring 58 within channel 50. In the locked orientation, as shown in FIGS. 6 and 8, locking element 54 is rotated and is fully translated though channel 50 such that end 66 engages with ring 58 to dispose ring 58 within groove 60, as described herein.

In some embodiments, engagement between head 20 and shaft 22 enables fastener 14 to have a selected movement of head 20 relative to vertebral tissue. In some embodiments, head 20 is selectively movable relative to shaft 22 through an angular range and disposable at a selected angle relative to shaft 22. In some embodiments, head 20 is selectively movable relative to shaft 22 through an angular range of 0-35 degrees. In some embodiments, the selected movement of head 20 includes rotation and/or pivotal movement of head 20 relative to shaft 22 about one or a plurality of axes. In some embodiments, the selected movement of head 20 includes rotation and/or pivotal movement of head 20 relative to shaft 22 through one or a plurality of planes. In some embodiments, the selected movement includes movement through one or more of transverse, vertical, horizontal, diagonal, coronal and/or sagittal planes of a body. In some embodiments, head 20 is selectively movable relative to shaft 22 in a fixed axis configuration.

In some embodiments, head 20 is attached with shaft 22 such that fastener 14 comprises, for example, a sagittal angulation screw, pedicle screw, monoaxial screw, facet screw, fixed screw, tissue penetrating screw, conventional screw, expanding screw, wedge, anchor, staple, nail and/or post.

In some embodiments, fastener 14 includes a head 120, as shown in FIGS. 1 and 9-12. Head 120 is configured for engagement with a shaft 22. In some embodiments, head 120 is selected from a plurality of alternate heads 120 and is configured for disposal with shaft 22 such that shaft 22 is interchangeable with the plurality of alternate heads 20, 120.

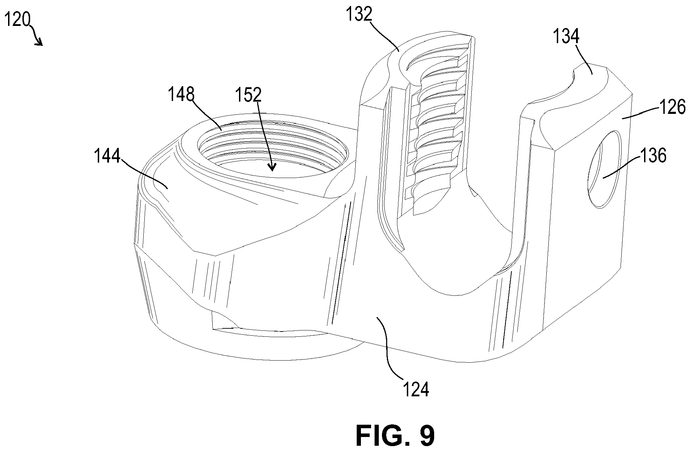

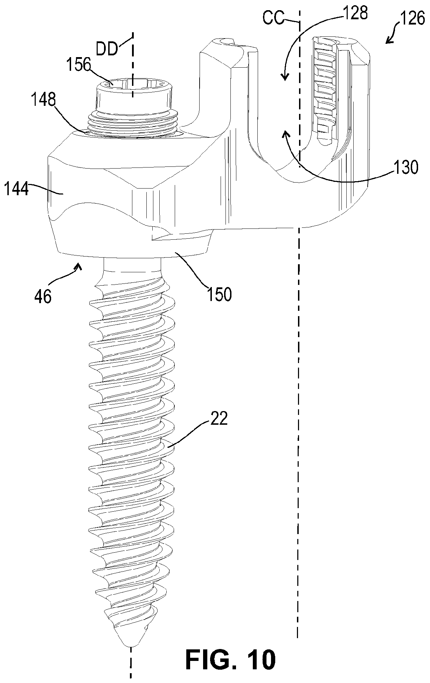

Head 120 includes a body 124, as shown in FIG. 9. Body 124 includes a surface that defines a rod receiver 126 configured to receive a spinal rod, including spinal rod 16 and/or spinal rod 18. In some embodiments, receiver 126 is configured to receive a spinal rod that is oriented medially relative to a spine and/or a spinal rod that is oriented laterally relative to a spine. Receiver 126 is disposed in an offset orientation relative to shaft 22, as shown in FIG. 10. Receiver 126 defines a longitudinal axis CC and is aligned along trajectory T2 or trajectory T3, similar to receiver 26, which is offset from the selected trajectory T1 of shaft 22, as shown in FIG. 1 and described herein. In some embodiments, all or only a portion of receiver 126 may have alternate cross section configurations, such as, for example, closed, V-shaped, ω-shaped, W-shaped, oval, oblong triangular, square, polygonal, irregular, uniform, non-uniform, offset, staggered, and/or tapered.

Referring to FIG. 10, an inner surface of receiver 126 defines a cavity 128 for disposal of spinal rod 16 or spinal rod 18, as shown in FIG. 1. Cavity 128 extends along longitudinal axis CC and includes a transverse passageway 130. In some embodiments, cavity 128 may have various cross section configurations, such as, for example, oval, oblong, triangular, rectangular, square, polygonal, irregular, uniform, non-uniform, variable and/or tapered. In some embodiments, all or a portion of the inner surface is smooth, rough, arcuate, undulating, mesh, porous, semi-porous, dimpled and/or textured.

Receiver 126 includes spaced apart arms 132, 134, as shown in FIG. 9. An outer surface of arm 134 defines a recess 136, as shown in FIG. 9. Recess 136 is configured to receive an insertion tool, compression instrument and/or surgical instruments for reducing spinal rods 16 or 18 with receiver 126 and/or to manipulate fastener 14. An inner surface of arms 132, 134 defines threads 138 for engagement with threads of a setscrew (not shown) to fix spinal rod 16 or spinal rod 18 with receiver 126. In some embodiments, arms 132, 134 may have the same or alternate cross section configurations, may be fabricated from a homogenous material or heterogeneously fabricated from different materials.

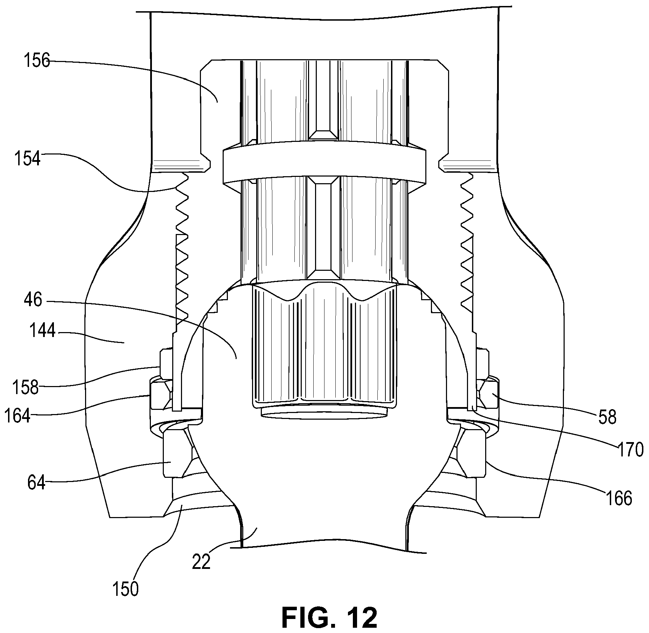

Body 124 includes a surface that defines a mount 144, as shown in FIG. 9. Mount 144 is configured for engagement with head 46 of shaft 22, as shown in FIG. 12. An outer surface of mount 144 defines an opening 148, an opening 150 and a longitudinal axis DD, as shown in FIGS. 9, 10 and 12. An inner surface of mount 144 defines a channel 152 disposed between openings 148, 150. Head 46 of shaft 22 is disposed within opening 150 and channel 152. In some embodiments, channel 152 may have various cross section configurations, such as, for example, oval, oblong, triangular, rectangular, square, polygonal, irregular, uniform, non-uniform, variable and/or tapered.

The inner surface of mount 144 defines a plurality of threads 154 for engagement with a threaded looking element 156, as shown in FIG. 12, similar to locking element 54 described herein. Locking element 156 is movable relative to mount 144 to dispose shaft 22 in a non-locked orientation and a locked orientation as described herein. The inner surface of mount 144 defines a circumferential upper groove 158 for disposal of ring 58, as shown in FIG. 12. Ring 58 is contractable in upper groove 158 and is engageable with an end 170 of locking element 156. The inner surface defines an expansion groove 164. The inner surface of mount 144 defines a circumferential lower groove 166 for disposal of ring 64. Ring 64 is expandable in expansion groove 164.

In some embodiments, spinal construct 12 includes head 220, as shown in FIG. 13. Head 220 is configured for engagement with a shaft 22. In some embodiments, head 220 is selected from a plurality of alternate heads 220 and is configured for disposal with a shaft 22 such that shaft 22 is interchangeable with the plurality of alternate heads 20, 120, 220.

Head 220 includes a body 224, as shown in FIG. 13. Body 224 includes a surface that defines a rod receiver 226 and a rod receiver 228. Receiver 226 is configured to receive a spinal rod, including spinal rod 16 and/or spinal rod 18. In some embodiments, receiver 226 is configured to receive a spinal rod that is oriented medially relative to a spine and/or a spinal rod that is oriented laterally relative to a spine. Receiver 226 is disposed in an offset orientation relative to shaft 22. Receiver 226 defines a longitudinal axis EE and is aligned along trajectory T2 or trajectory T3 that is offset from the selected trajectory T1 of shaft 22, as shown in FIG. 1 and described herein. In some embodiments, all or only a portion of receiver 226 may have alternate cross section configurations, such as, for example, closed, V-shaped, ω-shaped, W-shaped, oval, oblong triangular, square, polygonal, irregular, uniform, non-uniform, offset, staggered, and/or tapered.

An inner surface of receiver 226 defines a cavity 230 for disposal of spinal rod 16 or spinal rod 18, as shown in FIGS. 1 and 13. Cavity 230 extends along longitudinal axis EE and includes a transverse passageway 232. In some embodiments, cavity 230 may have various cross section configurations, such as, for example, oval, oblong, triangular, rectangular, square, polygonal, irregular, uniform, non-uniform, variable and/or tapered. In some embodiments, all or a portion of the inner surface is smooth, rough, arcuate, undulating, mesh, porous, semi-porous, dimpled and/or textured.

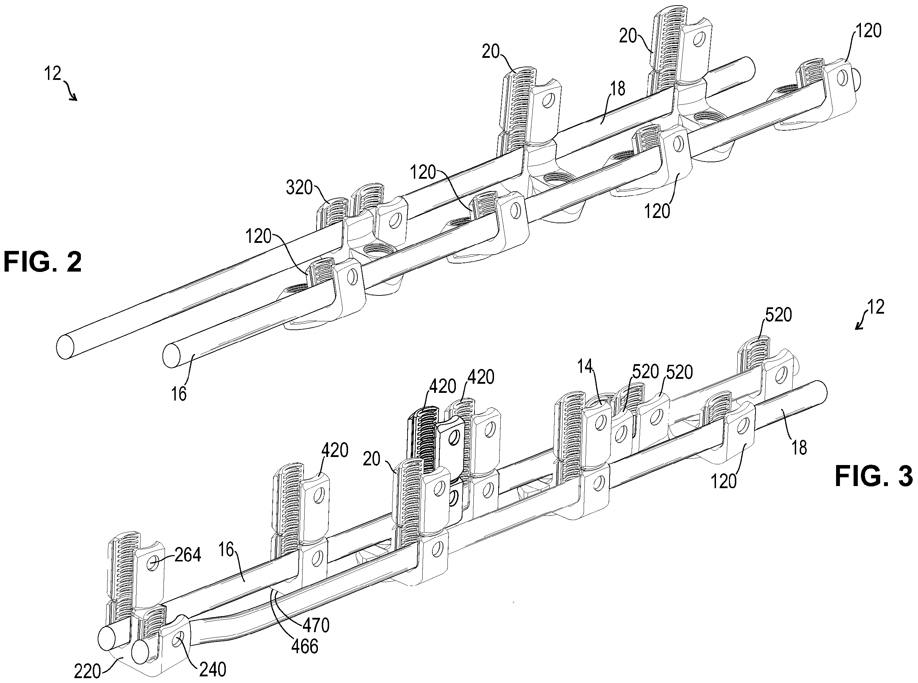

Receiver 226 includes spaced apart arms 234, 236, as shown in FIG. 13. An inner surface of arms 234, 236 defines threads 238 for engagement with threads of a setscrew (not shown). An outer surface of arm 236 defines a recess 240, as shown in FIG. 3. Recess 240 is configured to receive an insertion tool, compression instrument and/or surgical instruments for reducing spinal rods 16 or 18 with receiver 226 and/or to manipulate fastener 14.

Receiver 228 is configured to receive a spinal rod, including spinal rod 16 and/or spinal rod 18. In some embodiments, receiver 228 is configured to receive a spinal rod that is oriented medially relative to a spine and/or a spinal rod that is oriented laterally relative to a spine. Receiver 228 defines a longitudinal axis FF and receiver 228 along with shaft 22 are aligned along trajectory T1, as shown in FIG. 1 and described herein. In some embodiments, all or only a portion of receiver 228 may have alternate cross section configurations, such as, for example, closed, V-shaped, ω-shaped, W-shaped, oval, oblong triangular, square, polygonal, irregular, uniform, non-uniform, offset, staggered, and/or tapered.

An inner surface of receiver 228 defines a cavity 242 for disposal of spinal rod 16 or spinal rod 18, as shown in FIGS. 1 and 13. Cavity 242 extends along longitudinal axis FF and includes a transverse passageway 244. In some embodiments, cavity 242 may have various cross section configurations, such as, for example, oval, oblong, triangular, rectangular, square, polygonal, irregular, uniform, non-uniform, variable and/or tapered. In some embodiments, all or a portion of the inner surface is smooth, rough, arcuate, undulating, mesh, porous, semi-porous, dimpled and/or textured.

Receiver 228 includes spaced apart arms 246, 248, as shown in FIG. 13. An inner surface of arms 246, 248 defines threads 250 for engagement with threads of a setscrew (not shown) to fix spinal rod 16 or spinal rod 18 with receiver 228. In some embodiments, the setscrew is configured to temporarily lock with spinal rod 16 or spinal rod 18 to facilitate spinal rod 16 or spinal rod 18 seating with receiver 228. An outer surface of arm 246 defines a recess 252. Recess 252 is configured to receive an insertion tool, compression instrument and/or surgical instruments for reducing spinal rods 16 or 18 with receiver 228 and/or to manipulate fastener 14.

Arm 246 includes an extended reduction tab 254 and arm 248 includes an extended reduction tab 256, as shown in FIG. 13. Reduction tabs 254, 256 are configured to facilitate reduction of spinal rod 16 or spinal rod 18, as described herein, with receiver 228. Reduction tabs 254, 256 include break off portions 258, 260. In some embodiments, reduction tabs 254, 256 fracture and separate at break off portions 258, 260 at a predetermined force or torque limit, which may be in a range of approximately 2 Newton meters (N-m) to 8 Nm. Reduction tab 254 includes a recess 262 (shown in FIG. 13) and reduction tab 256 includes a recess 264 (shown in FIG. 3) configured to receive an insertion tool, compression instrument and/or surgical instruments for reducing rods 16 or 18 with receiver 228 and/or to manipulate fastener 14. In some embodiments, reduction tabs 254, 256 and arms 246, 248 may have the same or alternate cross section configurations, may be fabricated from a homogenous material or heterogeneously fabricated from different materials, and/or alternately formed of a material having a greater degree, characteristic or attribute of plastic deformability, frangible property and/or break away quality to facilitate fracture and separation of reduction tabs 254, 256 from arms 246, 248.

Body 224 includes a surface that defines an opening 266 and an inner surface defines a channel 270. Head 46 of shaft 22 is disposed with opening 266 and channel 270. In some embodiments, channel 270 may have various cross section configurations, such as, for example, oval, oblong, triangular, rectangular, square, polygonal, irregular, uniform, non-uniform, variable and/or tapered.

An inner surface of body 224 defines a circumferential upper groove (not shown), similar to upper groove 56 described above, for disposal of ring 58. Ring 58 is contractable in the upper groove. The inner surface defines an expansion groove (not shown), similar to expansion groove 60 described herein. Inner surface of body 224 defines a circumferential lower groove (not shown), similar to lower groove 62, for disposal of ring 64. Ring 64 is expandable in the expansion groove. Rings 58, 64 are configured to fix shaft 22 with head 220.

In some embodiments, spinal construct 12 includes a head 320, as shown in FIGS. 1 and 2. Head 320 is configured for engagement with a shaft 22. In some embodiments, head 320 is selected from a plurality of alternate heads 320 and is configured for disposal with a shaft 22 such that the shaft 22 is interchangeable with the plurality of alternate heads 20, 120, 220, 320.

Head 320 includes a body 324 that includes a surface that defines a rod receiver 326 and a rod receiver 328. Receivers 326, 328 are configured to receive a spinal rod, including spinal rod 16 and/or spinal rod 18. In some embodiments, receivers 326, 328 are configured to receive a spinal rod that is oriented medially relative to a spine and/or a spinal rod that is oriented laterally relative to a spine. Receivers 326, 328 are disposed in an offset orientation relative to shaft 22. Receivers 326, 328 are aligned along trajectory T3 that is offset from the selected trajectory T1, as shown in FIG. 1 and described herein. Body 324 includes a surface that defines a mount 344. Mount 344 is configured for engagement with head 46 of the shaft 22, as described herein.

In some embodiments, spinal construct 12 includes a head 420, as shown in FIGS. 1 and 3. Head 420 is configured for engagement a shaft 22. In some embodiments, head 420 is selected from a plurality of alternate heads 420 and is configured for disposal with a shaft 22 such that shaft 22 is interchangeable with the plurality of alternate heads 20, 120, 220, 320, 420.

Head 420 includes a body 424, as shown in FIG. 1. Body 424 includes a surface that defines a rod receiver 428. Receiver 428 is configured to receive a spinal rod, including spinal rod 16 and/or spinal rod 18. In some embodiments, receiver 428 is configured to receive a spinal rod that is oriented medially relative to a spine and/or a spinal rod that is oriented laterally relative to a spine. Receiver 428 along with shaft 22 are aligned along trajectory T1, as shown in FIG. 1 and described herein. Body 424 includes a surface that defines an opening 466 and an inner surface defines a channel 470, as shown in FIG 3 for disposal of head 46 of shaft 22 as described herein.

In some embodiments, spinal construct 12 includes a head 520, as shown in FIGS. 1 and 3. Head 520 is configured for engagement with a shaft 22. In some embodiments, head 520 is selected from a plurality of alternate heads 520 and is configured for disposal with a shaft 22 such that shaft 22 is interchangeable with the plurality of alternate heads 20, 120, 220, 320, 420, 520.

Head 520 includes a body 524, as shown in FIG. 1. Body 524 includes a surface that defines a rod receiver 528. Receiver 528 is configured to receive a spinal rod, including spinal rod 16 and/or spinal rod 18. In some embodiments, receiver 528 is configured to receive a spinal rod that is oriented medially relative to a spine and/or a spinal rod that is oriented laterally relative to a spine. Receiver 528 along with shaft 22 are aligned along trajectory T1, as shown in FIG. 1 and described herein. Body 524 includes a surface that defines an opening 566 and an inner surface defines a channel 570 for disposal of head 46 of shaft 22 as described herein.

In some embodiments, spinal implant system 10 can include one or a plurality of bone fasteners such as those described herein and/or fixation elements, which may be employed with a single vertebral level or a plurality of vertebral levels. In some embodiments, the bone fasteners may be engaged with vertebrae in various orientations, such as, for example, series, parallel, offset, staggered and/or alternate vertebral levels.

In some embodiments, spinal implant system 10 includes a spinal implant kit, as described herein, including the variously configured heads described herein, that are configured for selection from a plurality of alternate heads and are configured for disposal with a shaft 22 such that shaft 22 is interchangeable with the plurality of alternate heads, as described herein. In some embodiments, the spinal implant kit includes one or more components for disposal along a side of vertebrae of a patient to provide strength and facilitate surgical treatment.

In assembly, operation and use, spinal implant system 10, similar to the systems and methods described herein, can include a head 20, 120, 220, 320, 420 and/or 520 for connection with one or more shafts 22 and is employed with a surgical procedure for treatment of a spinal disorder affecting a section of a spine of a patient, as discussed herein. Spinal implant system 10 is employed with a surgical procedure for treatment of a condition or injury of an affected section of the spine.

In some embodiments, spinal implant system 10 comprises a spinal implant kit, which includes one or more selectable interchangeable heads, including the heads described herein, that are configured for connection with one or more selectable interchangeable shafts, including shaft 22, to facilitate disposal of fasteners 14 along a side of a vertebrae of a patient along a single bone fastener trajectory, as described herein. In some embodiments, the one or more selected interchangeable shafts, including shaft 22, interface with one or more selected interchangeable heads, including the heads described herein, to comprise one or more fasteners 14 and/or configurations. The components of fasteners 14 and one or a plurality of spinal implants, for example, spinal rod 16, 18 can be delivered or implanted as a pre-assembled device or can be assembled in situ. The components of spinal implant system 10 may be completely or partially revised, removed or replaced.

In some embodiments, a shaft 22 is selected from the kit of the one or more interchangeable shafts for interchangeable connection with a selected head described herein from the one or more interchangeable heads to comprise a fastener 14 having a selected movement relative to shaft 22.

In use, to treat a selected section of vertebrae V, as shown in FIG. 1, a medical practitioner obtains access to a surgical site including vertebrae V in any appropriate manner, such as through incision and retraction of tissues. In some embodiments, spinal implant system 10 can be used in any existing surgical method or technique including open surgery, mini-open surgery, minimally invasive surgery and percutaneous surgical implantation, whereby vertebrae V is accessed through a miniincision, or a sleeve that provides a protected passageway to the area. Once access to the surgical site is obtained, the particular surgical procedure can be performed for treating the spine disorder.

An incision is made in the body of a patient and a cutting instrument (not shown) creates a surgical pathway for implantation of components of spinal implant system 10. A preparation instrument (not shown) can be employed to prepare tissue surfaces of vertebrae V, as well as for aspiration and irrigation of a surgical region.

Shafts 22 are fixed with tissue to engage with vertebrae V along a single side S1 of vertebrae V. Each shaft 22 is manipulated to drive, torque, insert and/or align shafts 22 with vertebrae V along a selected trajectory T1, as shown in FIG. 1. Heads 20 are disposed with shafts 22 in a snap-fit assembly, such that heads 20 include lateral offset receivers 26, as shown in FIG. 1. Receivers 26 are disposed along a lateral portion of side S1 relative to vertebrae V. Ring 58 is disposed with upper groove 56 and ring 64 is disposed with lower groove 62 of each mount 44 in a contracted orientation, as shown in FIG. 7. Each head 20 is assembled with each shaft 22 by translating head 20, in a direction shown by arrow A in FIG. 7. Engagement of head 46 of shaft 22 with mount 44 via channel 50 causes a surface of head 46 to engage with ring 64 such that ring 64 is translated, in a direction shown by arrow B in FIG. 7, disposing ring 64 into expansion groove 60 in an expanded orientation. Head 46 translates further through mount 44 in the direction shown by arrow B in FIG. 7 and passes further through ring 64 as ring 64 is driven back into lower groove 62. Ring 64 resiliently contracts into its natural state around head 46.

Locking element 54 is rotated to translate locking element 54 in the direction shown by arrow A to position locking element 54 in the locked orientation. End 66 of locking element 54 engages ring 58 to dispose ring 58 into expansion groove 60 such that ring 58 resiliently opens into its natural orientation, as shown in FIG. 8. Ring 58 is oriented for abutting and/or contacting engagement with ring 64 to resist and/or prevent translation of ring 64 from lower groove 62 into expansion groove 60, and thus providing fixed connection of head 20 with shaft 22 including permanent capture of head 20 and shaft 22, as shown in FIG. 8.

Head 320, similar to head 20, is disposed with shaft 22 in a snap-fit assembly, such that head 320 includes lateral offset receivers 326, 328, as shown in FIG. 1. Receivers 326, 328 are disposed along a lateral portion of side S1 relative to vertebrae V. A spinal rod, for example, a lateral spinal rod 18 is delivered to the surgical site adjacent vertebrae V. Spinal rod 18 is disposed with receivers 26 of heads 20 and receivers 326, 328 of head 320 in an offset orientation relative to shafts 22 and are aligned along a trajectory T2, as shown in FIG. 1. Set screws (not shown) engage with a surgical instrument, such as, for example, a driver (not shown), which advances the set screws into engagement with receivers 26, 326 and 328 to lock spinal rod 18 with receivers 26, 326 and 328 to attach spinal rod 18 with vertebrae V.

Heads 120, similar to head 20 are disposed with shafts 22 in a snap-fit assembly, such that heads 120 include medial offset receivers 126, as shown in FIG. 1. Receivers 126 are disposed along a medial portion of side S1 relative to vertebrae V. A spinal rod, for example, a medial spinal rod 16 is delivered to the surgical site adjacent vertebrae V. Spinal rod 16 is disposed with receivers 126 of heads 120 in an offset orientation relative to shafts 22 and are aligned along a trajectory T3, as shown in FIG. 1. Set screws (not shown) engage with the driver which advances the set screws into engagement with receivers 126 to lock spinal rod 16 with receivers 126 to attach spinal rod 16 with vertebrae V.

Shafts 22 are fixed with tissue to engage with vertebrae V along a single side S2 of vertebrae V along a selected trajectory T1, as shown in FIG. 1. Heads 20 are disposed with shafts 22 in a snap-fit assembly described herein, such that heads 20 include lateral offset receivers 26, as shown in FIG. 1. Receivers 26 are disposed along a lateral portion of side S2 relative to vertebrae V. Head 120, similar to head 20, is disposed with shaft 22 in a snap-fit assembly, such that head 120 includes a lateral offset receiver 126, as shown in FIG. 1. Receiver 126 is disposed along a lateral portion of side S2 relative to vertebrae V. Head 220, similar to head 20, is disposed with shaft 22 in a snap-fit assembly, such that head 220 includes a lateral offset receiver 226, as shown in FIG. 1. Receiver 226 is disposed along a lateral portion of side S2 relative to vertebrae V.

A spinal rod, for example, a lateral spinal rod 18 is delivered to the surgical site adjacent vertebrae V. Spinal rod 18 is disposed with receivers 26, receiver 126 and receiver 226 of head 220 in an offset orientation relative to shafts 22 and are aligned along a trajectory T2, as shown in FIG. 1. Set screws (not shown) engage with the driver which advances the set screws into engagement with receivers 26, 126 and 226 to lock spinal rod 18 with receivers 26, 126 and 226 to attach spinal rod 18 with vertebrae V.

Heads 420, similar to head 20, are disposed with shafts 22 in a snap-fit assembly described herein, such that heads 420 include medial offset receivers 428, as shown in FIG. 1. Receivers 428 are disposed along a medial portion of side S2 relative to vertebrae V. Heads 520, similar to head 20, is disposed with shaft 22 in a snap-fit assembly, such that heads 520 include medial offset receivers 528, as shown in FIG. 1. Receivers 528 are disposed along a medial portion of side S2 relative to vertebrae V. Head 220 includes a medial offset receiver 228 that is disposed along a medial portion of side S2 relative to vertebrae V, as shown in FIG. 1.

A spinal rod, for example, a medial spinal rod 16 is delivered to the surgical site adjacent vertebrae V. Spinal rod 16 is disposed with receivers 428, receivers 528 and receiver 228 of head 220 in an orientation relative to shafts 22 and are aligned along a trajectory T3, as shown in FIG. 1. In some embodiments, trajectory T3 is the same trajectory T1. Set screws (not shown) engage with a driver which advances the set screws into engagement with receivers 428, 528 and 228 to lock spinal rod 16 with receivers 428, 528 and 228 to attach spinal rod 16 with vertebrae V.

In some embodiments, the spinal constructs of spinal implant system 10, as described herein, are fixed with vertebrae V in a side by side orientation and/or a bi-lateral arrangement to stabilize vertebrae V and affect growth for a correction treatment to treat spine pathologies, as described herein. In some embodiments, one or more of shafts 22, as described herein, are fixed with tissue, which may include selected vertebrae, for example, cervical, thoracic, lumber, sacral and/or iliac bone, one or more portions of vertebrae, for example, lamina, pedicle, spinous process, transverse process, cancellous and/or cortical bone surfaces, and/or ribs. In some embodiments, one or all of the components of spinal implant system 10 can be delivered or implanted as a pre-assembled device or can be assembled in situ, in a selected order of assembly or the order of assembly of the particular components of system 10 can be varied according to practitioner preference, patient anatomy or surgical procedure parameters.

Upon completion of the procedure, the surgical instruments, assemblies and non-implanted components of spinal implant system 10 are removed from the surgical site and the incision is closed. One or more of the components of spinal implant system 10 can be made of radiolucent materials such as polymers. Radiomarkers may be included for identification under x-ray, fluoroscopy, CT or other imaging techniques. In some embodiments, the use of surgical navigation, microsurgical and image guided technologies may be employed to access, view and repair spinal deterioration or damage, with the aid of spinal implant system 10.

In some embodiments, spinal implant system 10 includes an agent, which may be disposed, packed, coated or layered within, on or about the components and/or surfaces of spinal implant system 10. In some embodiments, the agent may include bone growth promoting material, such as, for example, bone graft to enhance fixation of the bone fasteners with vertebrae. In some embodiments, the agent may include one or a plurality of therapeutic agents and/or pharmacological agents for release, including sustained release, to treat, for example, pain, inflammation and degeneration.

It will be understood that various modifications may be made to the embodiments disclosed herein. Therefore, the above description should not be construed as limiting, but merely as exemplification of the various embodiments. Those skilled in the art will envision other modifications within the scope and spirit of the claims appended hereto.

For example, further embodiments of the invention are as follows:

According to a first further embodiment, there is provided a spinal implant system comprising: a first bone fastener including a first head and a first shaft, the first head including a medial rod receiver offset from the first shaft; a second bone fastener including a second head and a second shaft, the second head including a medial rod receiver offset from the second shaft; a third bone fastener including a third head and a third shaft, the third head including a lateral rod receiver offset from the third shaft; a fourth bone fastener including a fourth head and a fourth shaft, the fourth head including a lateral rod receiver offset from the fourth shaft, the shafts being configured for fixation with vertebral tissue in alignment along a selected trajectory of the vertebral tissue; a first spinal rod configured for disposal within the medial rod receivers; and a second spinal rod configured for disposal within the lateral rod receivers.

According to a second further embodiment, there is provided the spinal implant system of the first further embodiment, wherein the medial rod receivers are configured for alignment along a first trajectory offset from the selected trajectory.

According to a third further embodiment, there is provided the spinal implant system of the first or second further embodiment, wherein the lateral rod receivers are configured for alignment along a second trajectory offset from the selected trajectory.

According to a fourth further embodiment, there is provided the spinal implant system of one of the first to third further embodiments, wherein the first head is manually engageable with the first shaft to connect the first head and the first shaft in a non-instrumented snap-fit assembly.

According to a fifth further embodiment, there is provided a method for treating a spine, the method comprising the steps of: fixing a first shaft of a first bone fastener with vertebral tissue, the first bone fastener including a first head including a medial rod receiver offset from the first shaft; fixing a second shaft of a second bone fastener with the vertebral tissue in alignment with the first shaft along a selected trajectory of the vertebral tissue, the second bone fastener including a second head including a lateral rod receiver offset from the second shaft; disposing a first spinal rod within the medial rod receiver; and disposing a second spinal rod within the lateral rod receiver.

According to a sixth further embodiment, there is provided the method for treating a spine according to the fifth further embodiment, wherein the selected trajectory is disposed along a sagittal orientation of the vertebral tissue.

According to a seventh further embodiment, there is provided the method for treating a spine according to the fifth or sixth further embodiment, wherein the medial rod receiver is configured for alignment along a first trajectory offset from the selected trajectory and the lateral rod receiver is configured for alignment along a second trajectory offset from the selected trajectory.

## Claims

1. A spinal implant system comprising:
a first bone fastener including a first head and a first shaft, the first head including a medial rod receiver offset from the first shaft; and
a second bone fastener including a second head and a second shaft, the second head including a lateral rod receiver offset from the second shaft,
the shafts being configured for fixation with vertebral tissue in alignment along a selected trajectory of the vertebral tissue.

2. The spinal implant system as recited in Claim 1, further comprising a third bone fastener including a third head and a third shaft configured for fixation with the vertebral tissue in alignment along the selected trajectory, the third head including a medial rod receiver offset from the third shaft.

3. The spinal implant system as recited in Claim 2, wherein the medial rod receivers are configured for alignment along a first trajectory offset from the selected trajectory.

4. The spinal implant system as recited in Claim 2 or 3, further comprising a spinal rod configured for disposal within the medial rod receivers.

5. The spinal implant system as recited in one of Claims 2-4, further comprising a fourth bone fastener including a fourth head and a fourth shaft configured for fixation with the vertebral tissue in alignment along the selected trajectory, the fourth head including a lateral rod receiver offset from the fourth shaft.

6. The spinal implant system as recited in Claim 5, wherein the lateral rod receivers are configured for alignment along a second trajectory offset from the selected trajectory.

7. The spinal implant system as recited in Claim 5 or 6, further comprising a spinal rod configured for disposal within the lateral rod receivers.

8. The spinal implant system as recited in one of Claims 1-7, wherein the selected trajectory is disposed along a sagittal orientation of the vertebral tissue.

9. The spinal implant system as recited in one of Claims 1-8, wherein the first head is manually engageable with the first shaft to connect the first head and the first shaft in a non-instrumented snap-fit assembly.

10. The spinal implant system as recited in one of Claims 1-9, wherein the first head includes a first groove configured for disposal of a first resilient member that is contractable in the first groove, and a second groove configured for disposal of a second resilient member that is expandable in the second groove to connect the first head and the first shaft.

11. The spinal implant system as recited in one of Claims 1-10, wherein the first bone fastener includes a head that is selected from a plurality of alternately configured heads and is configured for disposal with the first shaft such that the first shaft is interchangeable with the plurality of alternately configured heads.

12. The spinal implant system as recited in Claim 11, wherein the plurality of alternately configured heads includes a head configured for fixed axial movement relative to the first shaft and a head configured for multi axial movement relative to the first shaft.

13. The spinal implant system as recited in one of Claims 1-12, wherein the first head has a first arm and a second arm that define the at least one medial rod receiver, the arms having extended reduction tabs including break off portions.
